# EUROPÄISCHE PATENTANMELDUNG

(11) **EP 1 143 240 A1**
(43) Veröffentlichungstag der Anmeldung: **10.10.2001**
(21) Anmeldenummer: 00103882.7
(22) Anmeldetag: 24.02.2000
(51) Int. Cl.: G01N 27/49

(54) **Verfahren und Vorrichtung zum Bestimmen von Charakteristika einer Probenflüssigkeit mit einer Mehrzahl von Substanzen**

(71) Anmelder: FRAUNHOFER-GESELLSCHAFT ZUR FÖRDERUNG DER ANGEWANDTEN FORSCHUNG E.V., 80636 München (DE)
(72) Erfinder: Müller, Rudolf, Prof. Dr., 82319 Starnberg (DE); Wabner, Dietrich, Prof. Dr., 85748 Garching (DE); Endres, Hanns-Erik, Dr., 80689 München (DE); Wurdack, Ilse, Dr., 80339 München (DE); Pfeiffer, Peter, Dr., 80809 München (DE)
(74) Vertreter: Schoppe, Fritz, Dipl.-Ing.

(57) **Zusammenfassung**

Das erfindungsgemäße Verfahren zum Bestimmen von Charakteristika einer Probenflüssigkeit mit einer Mehrzahl von Substanzen umfaßt das Aufnehmen von Strom-Spannungs-Meßdaten einer Flüssigkeit mit zumindest einer bekannten Charakteristik, das Transformieren der Meßdaten der Flüssigkeit in einen Merkmalsraum, um eine erste Mehrzahl von Merkmalswerten zu erhalten, das Aufnehmen von Strom-Spannungs-Meßdaten der Probenflüssigkeit, das Transformieren der Meßdaten der Probenflüssigkeit in den Merkmalsraum, um eine zweite Mehrzahl von Merkmalswerten zu erhalten, und das Bestimmen von zumindest einer Charakteristik der Probenflüssigkeit basierend auf den Merkmalswerten der Probenflüssigkeit bezogen auf die Merkmalswerte der Flüssigkeit mit der zumindest einen bekannten Charakteristik.

## Beschreibung

Diese Erfindung bezieht sich auf die Untersuchung von Flüssigkeiten, insbesondere von Körperflüssigkeiten, wie z.B. Harn, Liquor, usw. oder von flüssigen Lebensmitteln. Insbesondere bezieht sich die Erfindung auf das Gebiet der Harndiagnose.

Harnuntersuchungen sind im Stand der Technik bekannt. Da dieselben nicht invasiv sind, belasten sie den Patienten nicht, und jeder Zuwachs an Informationen aus solchen Untersuchungen ist von besonderem kommerziellen Interesse. Es stehen derzeit im wesentlichen zwei Verfahren zur Verfügung, um eine Harnflüssigkeit zu untersuchen. Bei dem ersten Verfahren werden Teststreifen verwendet, die mit bis zu 20 Chemikalien beschichtet sind und auf einen Kontakt mit bestimmten Substanzen hin farblich umschlagen. Diese Teststreifen werden in die zu untersuchende Harnprobe eingetaucht. Hiermit kann ohne großen Aufwand eine Beurteilung erfolgen, die aber im wesentlichen nur qualitativ ist, da für den Farbumschlag ein bestimmter Schwellenwert des zu bestimmenden Stoffes vorhanden sein muß. Bei einem zweiten Verfahren wird das Infrarotspektrum einer Harnprobe, die zuvor eventuell mit bestimmten Reagenzien versetzt wurde, aufgenommen und ausgewertet. Geräte, die diese Infrarotspektralanalysen durchführen können, erfordern jedoch eine erhebliche Investitionssumme von etwa 200.000 DM und werden deshalb hauptsächlich in Kliniken verwendet. Sowohl der gerätetechnische als auch der logistische Aufwand, um mehrere Harnproben für eine Spektralanalyse in ein Labor zu bringen, die Untersuchungen durchzuführen und die Ergebnisse den entsprechenden Harnproben zuzuordnen und dem entsprechenden Arzt zu übermitteln, erfordern einen erheblichen Aufwand, wie z.B. in der Buchhaltung, dem Transportwesen, usw. Obwohl eine solche Infrarotspektraluntersuchung typischerweise lediglich 20 Minuten dauert, vergehen, bis dem Arzt die Ergebnisse vorliegen, im Fall eines klinikinternen Labors einige Stunden und in dem Fall eines niedergelassenen Arztes mehrere Tage.

Für Untersuchungen bezüglich Flüssigkeiten aller Art sind im Stand der Technik ferner Meßeinrichtungen zur Aufnahme von Cyclovoltagrammen bekannt. Solche Meßeinrichtungen sind in der Lage, durch zyklisches Anlegen einer Spannungsrampe an eine Probenflüssigkeit und gleichzeitiges Messen des resultierenden Elektrodenstromes eine Strom-Spannungs-Charakteristik der Probenflüssigkeit aufzunehmen, die wiederum Auskunft über entsprechende Elektrodenvorgänge von Inhaltsstoffen der Probenflüssigkeit gibt. Bei dieser Untersuchungsart spricht man deshalb ferner von einer "elektrochemischen Spektroskopie". Die Elektrodenvorgänge, die zu der StromSpannungs-Charakteristik beitragen, umfassen Reduktions-, Oxidationsprozesse, vor- oder nachgelagerte chemische Reaktionen, Adsorptionen von Reaktanden oder Produkten, Elektrodenbelegungen, usw. Dieselben tragen additiv zu der Strom-Spannungs-Charakteristik, dem sogenannten Cyclovoltagramm, bei. Cyclovoltagramme liefern somit eine schnelle Übersicht für das Verhalten eines elektrochemischen Systems.

Die Auswertung der voltammetrisch gewonnenen Meßkurven erfordert jedoch bis jetzt den Fachmann, der aus den Kurvenformen typische Kurvenformen erkennen kann und daraus Schlüsse auf die vorhandenen Reaktionen und die im Substrat vorhandenen Stoffe zieht. Eine derartige Datenauswertung durch den Praktiker, bzw. Arzt, oder das Laborpersonal ist jedoch mit Sicherheit in den weitaus meisten Fällen nicht möglich, da sich die Auswirkungen der verschiedenen Elektrodenvorgänge auf das Cyclovoltagramm gegenseitig überlagern.

Die Aufgabe der vorliegenden Erfindung besteht darin, ein wenig aufwendiges Verfahren und eine wenig aufwendige Vorrichtung zum Bestimmen von Charakteristika einer Probenflüssigkeit mit einer Mehrzahl von Substanzen zu schaffen, die eine schnelle Bestimmung von Charakteristika einer Probenflüssigkeit ermöglichen.

Diese Aufgabe wird durch ein Verfahren gemäß Anspruch 1 und eine Vorrichtung gemäß Anspruch 16 gelöst.

Das erfindungsgemäße Verfahren zum Bestimmen von Charakteristika einer Probenflüssigkeit mit einer Mehrzahl von Substanzen umfaßt das Aufnehmen von Strom-Spannungs-Meßdaten einer Flüssigkeit mit zumindest einer bekannten Charakteristik, das Transformieren der Meßdaten der Flüssigkeit in einen Merkmalsraum, um eine erste Mehrzahl von Merkmalswerten zu erhalten, das Aufnehmen von Strom-Spannungs-Meßdaten der Probenflüssigkeit, das Transformieren der Meßdaten der Probenflüssigkeit in den Merkmalsraum, um eine zweite Mehrzahl von Merkmalswerten zu erhalten, und das Bestimmen von zumindest einer Charakteristik der Probenflüssigkeit basierend auf den Merkmalswerten der Probenflüssigkeit bezogen auf die Merkmalswerte der Flüssigkeit mit der zumindest einen bekannten Charakteristik.

Die erfindungsgemäße Vorrichtung zum Bestimmen von Charakteristika einer Probenflüssigkeit mit einer Mehrzahl von Substanzen umfaßt eine erste Aufnahmeeinrichtung zum Aufnehmen von Strom-Spannungs-Meßdaten einer Flüssigkeit mit zumindest einer bekannten Charakteristik und von Strom-Spannungs-Meßdaten der Probenflüssigkeit, eine erste Verarbeitungseinrichtung zum Transformieren der Meßdaten der Flüssigkeit in einen Merkmalsraum, um eine erste Mehrzahl von Merkmalswerten zu erhalten, und zum Transformieren der Meßdaten der Probenflüssigkeit in den Merkmalsraum, um eine zweite Mehrzahl von Merkmalswerten zu erhalten, und eine zweite Verarbeitungseinrichtung zum Bestimmen von zumindest einer Charakteristik der Probenflüssigkeit basierend auf den Merkmalswerten der Probenflüssigkeit bezogen auf die Merkmalswerte der Flüssigkeit mit der zumindest einen bekannten Charakteristik.

Gemäß einem Ausführungsbeispiel werden zum Bestimmen der zumindest einen Charakteristik einer Probenflüssigkeit eine Mehrzahl von Strom-Spannungs-Meßdaten einer Mehrzahl von Referenzflüssigkeiten aufgenommen. Diese Strom-Spannungs-Meßdaten entsprechen hierbei Cyclovoltagrammen, die durch zyklisches Anlegen einer Spannungsrampe in beiden Richtungen und gleichzeitiges Messen des Elektrolysestromes erhalten werden. Die resultierenden Meßdaten werden daraufhin einer mathematischen Operation unterzogen, wie z.B. einer Fourier-Transformationen, einer Wavelet-Transformation oder dergleichen. Aus den sich ergebenden "spektralen" bzw. transformierten Meßdaten wird ein Leistungsspektrum herausgeschnitten, um die Datenmenge für die nachfolgende Verarbeitung zu reduzieren. Aus diesen an Zahl reduzierten (von nun an einfach als "reduziert" bezeichneten) spektralen Meßdaten der Mehrzahl von Referenzflüssigkeiten wird mittels einer Hauptkomponentenanalyse eine Transformationsmatrix bestimmt, die die Meßdaten in einen niedrigdimensionalen Merkmalsraum abbildet. Hierauf werden Strom-Spannungs-Meßdaten einer Mehrzahl von Flüssigkeiten mit zumindest einer bekannten Charakteristik aufgenommen, einer Spektraltransformation unterzogen und mittels der Transformationsmatrix in den Merkmalsraum abgebildet, wobei sich eine erste Mehrzahl von Merkmalswerten ergibt. Dieselben Schritte werden durchgeführt, um Merkmalswerte der Probenflüssigkeit mit unbekannter Substanzzusammensetzung zu erhalten. Aus einem Vergleich der Merkmalswerte der Flüssigkeit mit zumindest einen bekannten Charakteristik und der Probenflüssigkeit mit der unbekannten Substanzzusammensetzung kann daraufhin die Probenflüssigkeit einer bestimmten Klasse, wie z.B. "Harnprobe eines Patienten, dem vor Probenentnahme kein Vitamin C verabreicht wurde", zugeordnet werden, oder ein bestimmter physikalischer Wert der Harnprobe, wie z.B. die Konzentration eines bestimmten Inhaltsstoffes, quantitativ erfaßt werden.

Die vorliegende Erfindung schließt folglich die Lücke zwischen den beiden im vorhergehenden erwähnten Verfahren, den Teststreifen und der Infrarotspektralanalyse. Im Gegensatz zu dem Einsatz von Teststreifen ist die vorliegende Erfindung in der Lage, quantitative Ergebnisse zu liefern. Überdies ist dies mit einem deutlich geringerem Aufwand möglich, als dies bei Infrarotspektralanalysen der Fall ist. Der geschätzte Gerätepreis für eine Realisierung der vorliegenden Erfindung liegt beispielsweise bei 20.000 DM am Anfang und bei einer größeren Serie bei etwa 5.000 DM und liegt somit deutlich unter den Anschaffungskosten von 200.000 DM für ein Infrarotspektralanalysegerät. Die Messung und Beurteilung der Proben kann vor Ort, wie z.B. bei einem niedergelassenen Arzt, und unmittelbar erfolgen, wobei die typische Meßdauer bei etwa 1-2 Minuten liegt. Folglich wird ferner das Risiko einer nicht kontrollierten Veränderung der Probe, wie z.B. eine Entmischung der Probe, langsame chemische Reaktionen und eine Beeinflussung durch Lichteinwirkung und Temperaturschwankungen, als Folge eines nicht definierten Transportes bzw. einer Lagerung vermieden.

Da sich die vorliegende Erfindung von den im vorhergehenden erwähnten Verfahren grundsätzlich unterscheidet, können darüber hinaus Ergebnisse erhalten werden, die als Ergänzung zu den herkömmlichen Verfahren verwendet werden können.

Die Anwendbarkeit der vorliegenden Erfindung ist überdies nicht auf Harnuntersuchungen beschränkt, sondern sie kann bei Flüssigkeiten aller Art, wie z.B. bei anderen Körperflüssigkeiten, flüssigen Lebensmitteln, Waschflüssigkeiten (Waschflotte), usw., eingesetzt werden.

Bevorzugte Ausführungsbeispiele der vorliegenden Erfindung werden nachfolgend bezugnehmend auf die beiliegenden Zeichnungen näher erläutert. Es zeigen:
- Fig. 1: schematisch den Aufbau einer Meßeinrichtung zur Aufnahme von Cyclovoltagrammen, wie sie bei der vorliegenden Erfindung verwendet werden kann;
- Fig. 2: ein Cyclovoltagramm, wie es sich aus einer Messung einer Harnprobe mittels einer Goldelektrode ergibt;
- Fig. 3a: den ersten Teil eines Flußdiagramm, das die Schritte eines Ausführungsbeispiels des erfindungsgemäßen Verfahrens beschreibt;
- Fig. 3b: den zweiten Teil des Flußdiagramms von Fig. 3a.
- Fig. 4: mehrere Cyclovoltagramme von Proben, die zu verschiedenen Zeitpunkten vor und nach einer Verabreichung bzw. einer Zugabe von Vitamin C entnommen wurden;
- Fig. 5: einen Merkmalsraum, der durch mittels einer Hauptkomponentenanalyse erhaltene Eigenvektoren aufgespannt wird, wobei Merkmalswerte eingetragen sind, die den Cyclovoltagrammen aus Fig. 3 entsprechen; und
- Fig. 6: eine Auftragung von Zeitwerten, die für vier zu verschiedenen Zeitpunkten entnommene Harnflüssigkeiten gemäß der Erfindung ermittelt wurden und die Zeitdauern zwischen Entnahme und Verabreichung von Vitamin C angeben, aufgetragen gegen die tatsächlichen Zeitdauern.

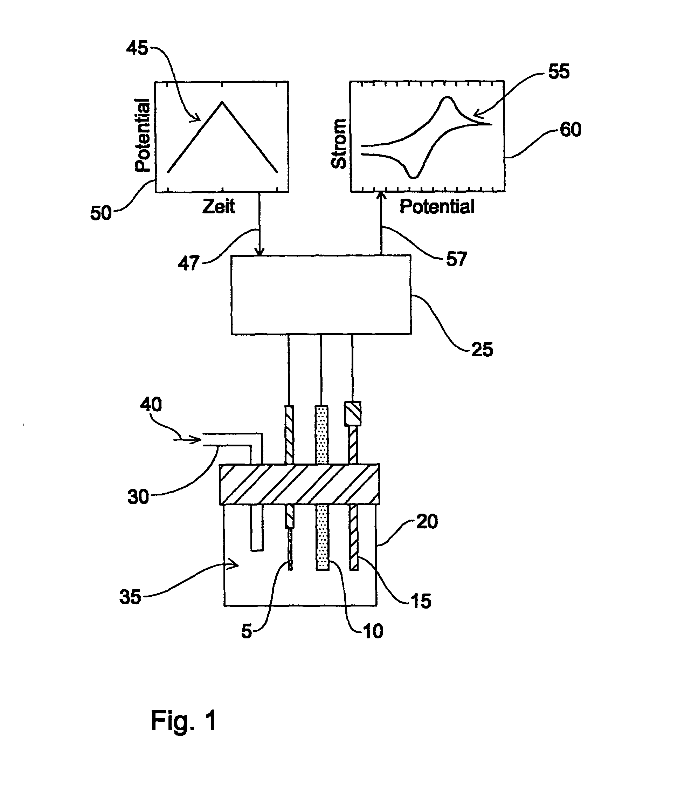

Es wird zunächst auf Fig. 1 Bezug genommen, die eine Vorrichtung zur Aufnahme von Strom-Spannungs-Meßdaten zeigt. Bei dem dargestellten Ausführungsbeispiel ist die Vorrichtung eine Vorrichtung zur Erzeugung eines Cyclovoltagramms einer Probenflüssigkeit. Diese Aufnahmeeinrichtung bzw. Meßeinrichtung zur Aufnahme von Cyclovoltagrammen besteht im wesentlichen aus drei Elektroden, nämlich einer Gegenelektrode 5, einer Arbeitselektrode 10 und einer Referenzelektrode 15, einer Meßkammer 20, in die sich die drei Elektroden 5, 10, 15 erstrecken, und einem Potentiostat 25, der eine Spannungsquelle und ein Strommeßgerät (nicht gezeigt) aufweist und mit den drei Elektroden 5, 10, 15 verbunden ist. Es ist ferner eine Begasungseinrichtung 30, wie z.B. ein Rohr, vorgesehen, durch das ein Inertgas, wie z.B. Stickstoff oder Argon, in eine in der Meßkammer 20 enthaltene Flüssigkeit 35, wie z.B. eine Probenflüssigkeit oder eine Kalibrierflüssigkeit, eingebracht werden kann, wie es durch einen Pfeil 40 angezeigt ist, um wahlweise in der Flüssigkeit 35 enthaltenen Sauerstoff auszutreiben. Es ist ebenfalls eine geeignete Vorrichtung zum Einbringen der Flüssigkeit 35 in die Meßkammer 20, wie z.B. ein am Boden der Meßkammer endendes Rohr, vorgesehen, ist aber aus Übersichtlichkeitsgründen nicht gezeigt.

Im folgenden wird nun die Funktionsweise der Meßeinrichtung erklärt. Über den Potentiostat 25 wird zwischen der Gegenelektrode 5 und der Arbeitselektrode 10 eine veränderliche Spannung angelegt, die in den Potentiostat 25 eingegeben werden kann, wie es durch einen Pfeil 47 gezeigt ist. Mittels der stromlosen Referenzelektrode 15, die vorzugsweise in der Nähe der Arbeitselektrode 10 angeordnet ist, wird hierzu ein definiertes Referenzpotential für die Arbeitselektrode 10 vorgegeben. Der Potentialverlauf 45, d.h. die Potentialänderung in Abhängigkeit von der Zeit, wird durch den Potentiostat 25 zwischen der Arbeitselektrode 10 und der stromlosen Referenzelektrode 15 vorgegeben. Der Potentialverlauf 45 ist in einer exemplarischen Auftragung 50 gezeigt, wobei das Potential über der Zeit aufgetragen ist. Wie es zu sehen ist, entspricht der Potentialverlauf 45 einer zyklischen Wiederholung eines sägezahnförmigen Signalverlaufes, bzw. dem zyklischen Durchlaufen einer Potentialrampe in beiden Richtungen, d.h. von einem negativen zu einem positiven Potential und umgekehrt. Der Potentiostat 25 mißt ferner den Strom, der zwischen der Gegenelektrode 5 und der Arbeitselektrode 10 fließt. Der Potentiostat 25 gibt den gemessenen Stromverlauf als Strom-Spannung-Meßdaten bzw. als Cyclovoltagramm 55 aus (Pfeil 57), wie exemplarisch bei 60 gezeigt ist, wo der Strom über dem Potential (der Spannung) aufgetragen ist.

Es wird darauf hingewiesen, daß, obwohl es in Fig. 1 nicht ersichtlich ist, die Gegenelektrode 5 vorzugsweise groß verglichen zu der Arbeitselektrode 10 ist, damit ausschließlich die elektrochemischen Vorgänge an der Arbeitselektrode 5 begrenzend auf den gemessenen Stromfluß wirken. Die aktive Fläche der Gegenelektrode ist beispielsweise um den Faktor 50, mindestens jedoch um den Faktor 2 größer als die Arbeitselektrode.

Obwohl im vorhergehenden beschrieben wurde, daß die Probenflüssigkeit 35 in die Meßkammer 20 eingebracht wird, ist es ferner möglich, die drei Elektroden 5, 10, 15 in die Probenflüssigkeit 35 einzutauchen. Darüber hinaus ist bei der letztgenannten Implementierung möglich, die Elektroden als eine Sonde zu implementieren, die über eine geeignete Schnellwechslervorrichtung als Einmalsonde verwendbar ist. Um die Signale von den Elektroden zu dem Potentiostat zu übertragen, kann eine solche Sonde ferner einen integrierten Vorverstärker zur Stromverstärkung aufweisen.

Es wird ferner darauf hingewiesen, daß eine Vorrichtung, die Temperaturschwankungen vermeidet, oder die eine definierte Temperatur an den Elektroden einstellt, d.h. eine Thermostatisierung, vorgesehen sein kann, da die an den Elektroden stattfindenden Reaktionen ferner von der Temperatur abhängig sein können.

Es wird ebenfalls darauf hingewiesen, daß für das Elektrodenmaterial verschiedene Materialien möglich sind, wie z.B. Platin, Gold oder Graphit. Wesentlich ist lediglich, daß das Elektrodenmaterial inert gegenüber den auftretenden chemischen Prozessen ist, um eine ausreichende Stabilität zu erzielen.

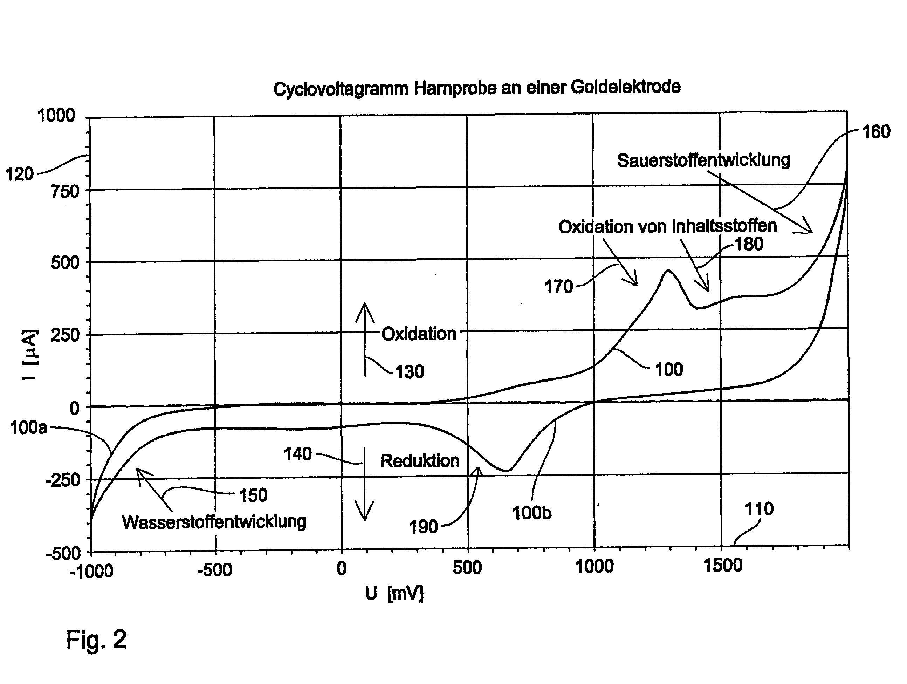

Es wird nun auf Fig. 2 Bezug genommen, die ein Cyclovoltagramm zeigt, das durch die Meßeinrichtung von Fig. 1 aufgenommen wurde. Als Probenflüssigkeit diente in diesem Fall eine Harnprobe, wobei als Elektrodenmaterial Gold verwendet wurde. Fig. 2 zeigt ein Cyclovoltagramm, bei dem auf der x-Achse 110 die angelegte Spannung bzw. das Potential U in mV und auf der y-Achse 120 die gemessene Stromstärke I in *µ*A aufgetragen ist. Wie es durch Pfeile 130 und 140 dargestellt ist, entsprechen negative Ströme Reduktionsvorgängen, während positive Ströme Oxidationsvorgängen entsprechen. Da eine Harnprobe als wesentlichen Bestandteil Wasser beinhaltet, wird der Potentialbereich für das Cyclovoltagramm, d.h. das Potentialfenster, durch die Wasserstoffentwicklung bei niedrigen Potentialen und die Sauerstoffentwicklung bei hohen Potentialen bestimmt. In der Figur ist der Potentialbereich beginnender Wasserstoffentwicklung in dem Cyclovoltagramm 100 durch einen Pfeil 150 und der Potentialbereich beginnender Sauerstoffentwicklung durch einen Pfeil 160 angezeigt. Bei dem vorliegenden wässrigen System, d.h. der Harnprobe, liegen diese Potentiale bei ca. -1000 mV bzw. +1100 mV.

Innerhalb dieses Potentialfensters können oxidierbare bzw. reduzierbare Wasserinhaltsstoffe in der Harnprobe bei bestimmten Potentialen elektrochemisch umgesetzt werden. Durch diese Prozesse werden Stromflüsse verursacht, die durch den Potentiostaten 25 (Fig. 1) gemessen werden und in dem Cyclovoltagramm 100 als Peak 170 bzw. 180 sichtbar werden. Da unterschiedliche Inhaltsstoffe der Probenflüssigkeit bei unterschiedlichen Potentialen oxidiert und reduziert werden, kann über die Lage der Strompeaks, d.h. bei welchem Potential der Strompeak auftritt, eine Aussage über die Art des Inhaltsstoffes getroffen werden. Darüberhinaus gibt die Höhe des Peaks 170, 180, d.h. die Stromstärke, die an dem Potential vorliegt, bei dem der Strompeak auftritt, Auskunft über die Konzentration des Stoffes.

Wie es zu sehen ist, weist das Cyclovoltagramm 100 zu jedem Potentialwert zwei Stromwerte auf, so daß sich das Cyclovoltagramm 100 in einen oberen Zweig 100a bzw. einen unteren Zweig 100b zusammensetzt. Hierbei entspricht der obere Zweig 100a den gemessenen Stromwerten während des linearen Potentialanstiegs, und der untere Zweig 100b den gemessenen Stromwerten während des linearen Potentialabfalls. Nähert sich das Potential während des Potentialanstiegs dem Oxidationspotential eines bestimmten Inhaltsstoffes, so steigt die gemessene Stromstärke an. Als Folge verringert sich die Oberflächenkonzentration des reagierenden Inhaltsstoffes an der entsprechenden Elektrode, d.h. der Arbeitselektrode, bei weiterem Potentialanstieg, und es setzt gleichzeitig ein Wachstum der Diffusionsschicht ein. Nach dem Erreichen eines maximalen Reaktionsstromes, wie z.B. bei 170, nimmt der Konzentrationsgradient und damit die Geschwindigkeit der elektrochemischen Reaktion bzw. die Stromstärke wieder ab, wodurch sich ein entsprechender Oxidationspeak ergibt (wie bei 170 und 180, wobei diese Peaks durch die Sauerstoffentwicklung 160 überlagert sind). Bei Durchlaufen dieses Potentials in entgegengesetzter Richtung wird, falls der entsprechende Prozeß reversibel ist, der Oxidationsprozeß umgekehrt, d.h. es findet eine Reduktion statt. Die Strom-bzw. Potential-Werte des resultierenden Oxidations- bzw. Reduktionspeaks, z.B. 170, geben Auskunft über die Reversibilität (Peakstromdifferenz) und das Reduktionspotential (Potentialdifferenz) des entsprechenden Inhaltsstoffes. Bei der vorliegenden Probenflüssigkeit (Harn) scheinen die entsprechenden Elektrodenreaktionen der Inhaltsstoffe nicht reversibel zu sein. Es wird darauf hingewiesen, daß die genaue Ausprägung des Peaks, d.h. Peakstromwert, Peakbreite usw., von der Scangeschwindigkeit abhängt, d.h. der Steigung der Potentialrampe. Ferner ist ein bei 190 zu erkennender Peak hauptsächlich das Ergebnis eines Deckschichtphänomens und hängt von dem verwendeten Elektrodenmaterial ab. In dem vorliegenden Fall von Gold als Elektrodenmaterial wird der Peak 190 durch eine Goldoxidreduktion bewirkt.

Wie es zu sehen ist, ist Harn jedoch ein sehr komplexes Medium mit sehr vielen verschiedenen Inhaltsstoffen, so daß sich in dem Cyclovoltagramm 100 einer Harnprobe sehr viele Peaks 170, 180 überlagern. Der Grund hierfür besteht darin, daß einerseits mehrere dieser Inhaltsstoffe bei sehr eng beieinanderliegenden Potentialen oxidiert bzw. reduziert werden, andererseits aber nur der gesamte dabei verursachte Stromfluß gemessen wird.

Bezüglich der chemisch-physikalischen Vorgänge bei der Cyclovoltammetrie und den Zusammenhängen zwischen physikalischen Größen und dem Cyclovoltagrammverlauf wird auf das Buch "Elektrochemie" von C. H. Hamann und W. Vielstich, Weinheim 1998, das im Wiley-VCH Verlag erschienen ist, und auf den Artikel "Cyclovoltammetrie - die Spektroskopie des Elektrochemikers" von J. Heinze in Angewandte Chemie, Bd. 96, 1984, S. 823-916 verwiesen, die hiermit durch Bezugnahme aufgenommen werden.

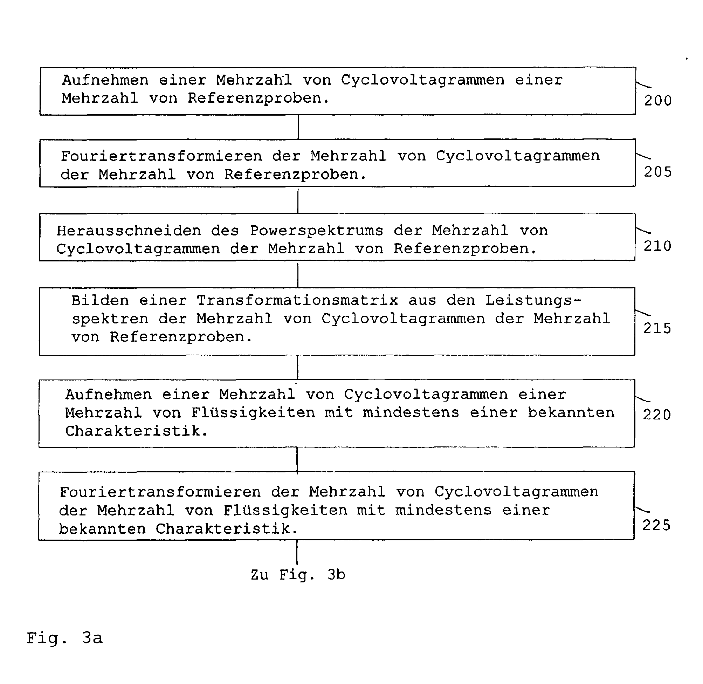

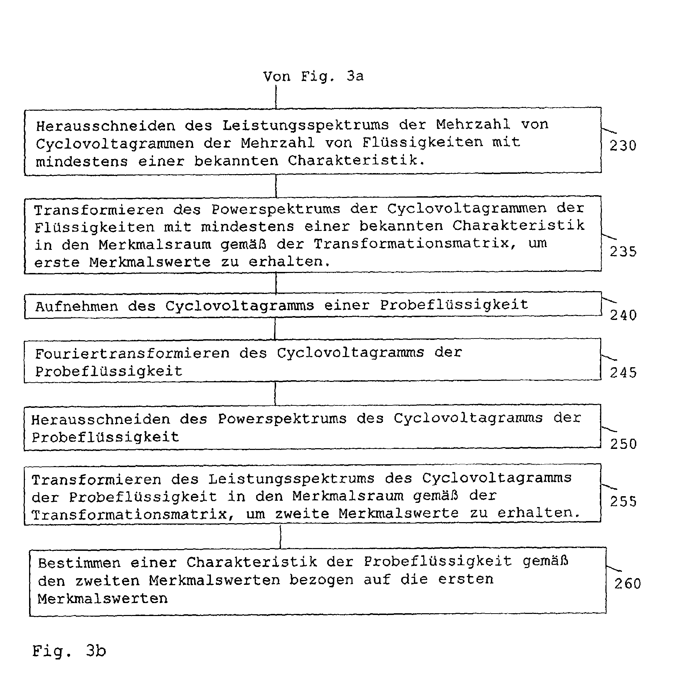

Bezugnehmend auf Fig. 3 wird nun ein Ausführungsbeispiel eines erfindungsgemäßen Verfahrens zum Bestimmen von Charakteristika einer Probenflüssigkeit mit einer Mehrzahl von Substanzen beschrieben. In einem Schritt 200 wird eine Mehrzahl von Cyclovoltagrammen einer Mehrzahl von Flüssigkeiten aufgenommen, die geeignet sind, um als Referenzflüssigkeiten zu dienen. In dem Fall einer Harnuntersuchung sind diese Referenzflüssigkeiten Harnproben von Normal-Testpersonen, d.h. von Personen, die in Hinblick auf die Gesundheit als normal eingestuft werden. Dies können ferner Personen sein, denen vor der Entnahme der Harnproben keine zusätzlichen Stoffe verabreicht wurden. Diese Voltagramme liegen dann als Meßvektoren vor. Bezüglich dieser Meßvektoren wird in einem Schritt 205 ein mathematischer Operator angewendet, wie z.B. eine Fourier-Transformation, eine Wavelet-Transformation, usw. Die erhaltenen spektralen Meßvektoren weisen ebenso viele Einträge auf wie die Meßvektoren, die im Schritt 200 aufgenommen wurden. Um die im folgenden zu verarbeitende Datenmenge zu reduzieren, wird in einem Schritt 210 aus den spektralen Meßvektoren vorzugsweise ein Leistungsspektrum herausgeschnitten, d.h. ein Feld von aufeinanderfolgenden Einträgen der spektralen Meßvektoren, deren Summe einen bestimmten Prozentsatz der Gesamtsumme aller Einträge der spektralen Meßvektoren überschreitet, herausgegriffen.

Diese "reduzierten" spektralen Meßvektoren werden in einem Schritt 215 einer Hauptkomponentenanalyse unterzogen, wie sie im Stand der Technik bekannt ist und beispielsweise in dem im Vieweg-Verlag erschienenen Buch "statistische Datenanalyse" von Werner A. Stahel, S. 307ff., beschrieben ist. Durch die Hauptkomponentenanalyse wird eine Transformationsmatrix ermittelt, die die reduzierten spektralen Meßvektoren in ein niedrigdimensionales Koordinatensystem bzw. einen Merkmalsraum transformiert. Hierzu werden aus den reduzierten spektralen Meßvektoren eine Kovarianzmatrix und die dazugehörigen Eigenvektoren und Eigenwerte bestimmt. Die Anzahl und die Größe der Eigenwerte sind ein Maß für die Anzahl der Merkmale, die aus den Meßwerten, die in Schritt 200 ermittelt wurden, extrahiert werden können, da viele der Meßwerte redundant sein können und somit allenfalls einen marginalen Beitrag zum Eigenvektorsystem liefern. Die Transformationsmatrix wird derart bestimmt, daß dieselbe einer Abbildungsvorschrift von reduzierten spektralen Meßvektoren in den Merkmalsraum entspricht, und daß der Merkmalsraum von denjenigen Eigenvektoren aufgespannt wird, deren Eigenwerte einen empirisch vorbestimmten Schwellenwert überschreiten. Der Schritt 215 stellt somit sicher, daß diese Abbildungsvorschrift an die zu messenden Probenflüssigkeiten, z.B. Harn, angepaßt ist. Der Schwellenwert kann eingestellt werden, um bezüglich der anschließenden Auswertung von Probenflüssigkeiten eine ausreichend hohe statistische Sicherheit sicherzustellen.

Nachdem im Schritt 215 die Abbildungsvorschrift bestimmt worden ist, um die reduzierten spektralen Meßvektoren in den Merkmalsraum abzubilden, werden die Schritte 200, 205 und 210 in Schritten 220, 225 bzw. 230 bezüglich einer Flüssigkeit wiederholt, von der zumindest eine Charakteristik bekannt ist. Diese Charakteristik kann beispielsweise die Konzentration eines bestimmten Inhaltsstoffes der Flüssigkeit umfassen, oder lediglich eine qualitative Aussage über die Flüssigkeit sein, wie z.B. daß dieselbe ein bestimmtes Verfallsdatum überschritten hat, oder daß dieselbe auf eine bestimmte Weise behandelt worden ist, z.B. mit Vitamin C versetzt worden ist. In einem Schritt 235 wird dann mittels der in dem Schritt 215 bestimmten Transformationsmatrix aus einem aufgenommenen Cyclovoltagramm der Flüssigkeit mit der zumindest einen bekannten Charakteristik ein erster Merkmalspunkt in dem Merkmalsraum bestimmt. Die Schritte 220, 225, 230 und 235 können ferner für mehrere Flüssigkeiten durchgeführt werden, wobei sich hierbei mehrere Merkmalspunkte ergeben.

In Schritten 240, 245, 250 und 255 werden daraufhin die Schritte 220, 225, 230 und 235 für die zu untersuchende Probenflüssigkeit, von der keine Charakteristik bekannt ist, wiederholt, wobei sich ein zweiter Merkmalspunkt ergibt.

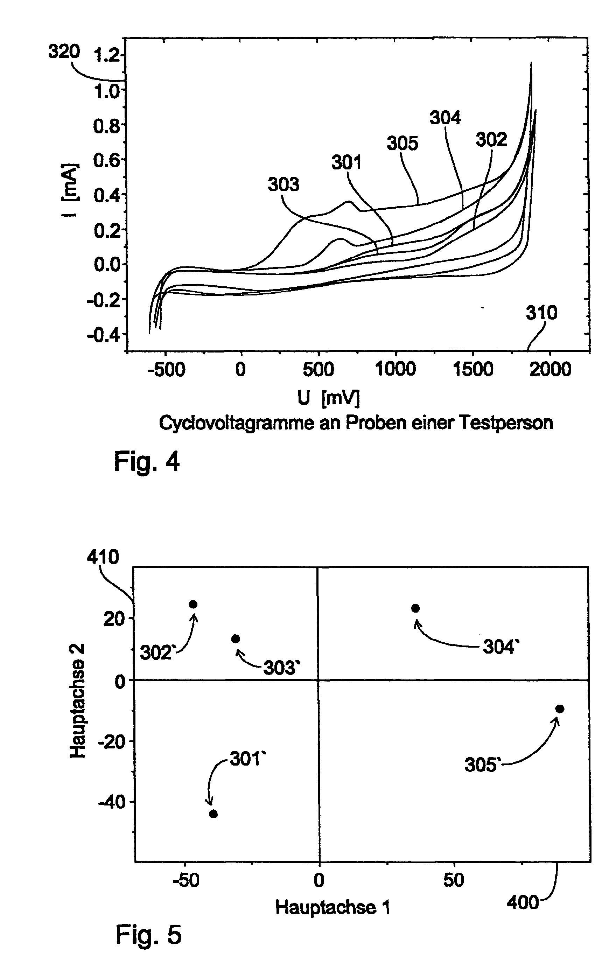

Der in Schritt 255 erhaltene Merkmalspunkt bzw. die erhaltenen Merkmalswerte (für jede Dimension des Merkmalraums einer) werden daraufhin in einem Schritt 260 entweder qualitativ einer bestimmten Klasse, die einer bestimmten Charakteristik entspricht, oder quantitativ einem bestimmten Wert zugeordnet, wie es bezugnehmend auf Fig. 4, 5 und 6 näher erläutert wird. Diese Zuordnung wird durchgeführt, indem die zweiten Merkmalswerte mit den ersten Merkmalswerten verglichen werden, die aus Cyclovoltagrammen von Proben extrahiert wurden, die zumindest eine bekannte Charakteristik aufweisen. Auf Grundlage von Merkmalswerten von Körperflüssigkeiten von Testpersonen mit einem bekannten Krankheitszustand kann eine Klassenzuordnung beispielsweise das Bestimmen einer Krankheit der Testperson bedeuten, der die entsprechende Probenflüssigkeit, z.B. Harn, Liquor, usw., entnommen wurde. Auf der Grundlage von Merkmalswerten von Proben mit einer bekannten Substanzzusammensetzung kann die Bestimmung eines quantitativen Werts beispielsweise die Bestimmung der Konzentration einer Inhaltsstoffes oder dergleichen sein.

Es wird darauf hingewiesen, daß es möglich ist, in den Schritten 205 und 220 dieselben Cyclovoltagramme zu verwenden. Es ist ferner möglich die Schritte 205, 210, 225, 230, 245 und 250 wegzulassen, und stattdessen die Schritte 215, 235, 255 direkt auf die Cyclovoltagramme anzuwenden. Zur Klarheit wird ferner darauf hingewiesen, daß die in Schritt 250 bestimmte Charakteristik stets auf eine Eigenschaft, wie z.B. eine Konzentration, ein Krankheitszustand, usw., bezogen ist, auf die sich die zumindest eine bekannte Charakteristik der Flüssigkeit von Schritt 220 bezieht.

Da die Deckschichtphenomäne (siehe 190 in Fig. 2) von dem verwendeten Eletrodenmaterial abhängen (der Peak bei 190 ist wie bereits erwähnt ein Effekt des Deckschichtphenomäns und nicht ein dem Oxidationspeak 170 entsprechender Reduktionspeak), und somit auch jeder Cyclovoltagrammverlauf von dem verwendeten Elektrodenmaterial abhängt, kann es vorteilhaft sein, bei den Aufnahmen der Cyclovoltagramme bei den Schritten 200, 220 und 240 das selbe Elektrodenmaterial zu verwenden. Es ist ferner möglich die Schritte 200, 220 und 240 mehrfach durchzuführen, so daß für jede Flüssigkeit Cyclovoltagramme unter Verwendung verschiedener Elektrodenmaterialien erhalten werden, also beispielsweise jede Cyclovoltagrammmessung mit Gold-, Platin und Graphit als Elektrodenmaterial durchgeführt wird. Die sich ergebenden Cyclovoltagramme für eine Flüssigkeit könnten hierauf für die anschließende Verarbeitung zu einem Meßvektor zusammengefaßt werden. Der Vorteil besteht darin, daß die Deckschichtphenomäne zusätzliche Informationen über die jeweiligen Flüssigkeiten liefern, die bei dem Verfahren von Fig. 3 zu besseren Ergebnissen führen können.

Ein weiterer Einstellungsparameter, der bei den Aufnahmen der Cyclovoltagramme berücksichtigt werden kann, ist die Scangeschwindigkeit. Da die Scangeschwindigkeit die genaue Form der Oxidations- und Reduktionspeaks beeinflußt, hängt der Cyclovoltagrammverlauf von der zur Aufnahme verwendeten Scangeschwindigkeit ab. Aus diesem Grund kann es vorteilhaft sein, für die Schritte 200, 220, 240 auch die Scangeschwindigkeit gleich zu wählen. Wiederum kann es vorteilhaft sein, jede Cyclovoltagrammaufnahme unter Verwendung verschiedener Scangeschwindigkeiten durchzuführen und die sich ergebenden Cyclovoltagramme zu einem Meßvektor zusammenzufassen. Hierdurch könnten weitere Informationen über die Flüssigkeiten aus den Diffusionsvorgängen und Durchtrittsreaktionen an der Elektroden erhalten und für die anschließende Auswertung verwendet werden.

Es wird ferner darauf hingewiesen, daß es insbesondere in dem Fall von Körperflüssigkeiten vorteilhaft sein kann, die verschiedenen Flüssigkeiten vor der Durchführung der Schritte 200, 220 und 240 durch Verdünnen auf einen gleichen Leitfähigkeitswert einzustellen. In dem Fall von Harnproben könnte es ansonsten vorkommen, daß die Harnproben von Patienten unterschiedlich konzentriert sind, abhängig von der Menge an Flüssigkeit, die der Patient vor Entnahme einer Probe getrunken hat. Da die Peakstromhöhe von der Konzentration der Inhaltsstoffe abhängt, hängt der Cyclovoltagrammverlauf von der Konzentration ab. Durch Einstellen aller Flüssigkeiten auf den selben Leitfähigkeitswert vor einer Cyclovoltagrammaufnahme kann können die erhaltenen Cyclovoltagramme standardisiert werden.

In folgenden wird nun auf Fig. 4 Bezug genommen, in der fünf Cyclovoltagramme 301, 302, 303, 304 und 305 gezeigt sind, die durch die Meßeinrichtung von Fig. 1 bezüglich Harnproben gemessen wurden, die einer Testperson zu verschiedenen Zeitpunkten nach Verabreichung von Vitamin C bzw. vor Verabreichung von Vitamin C entnommen wurden, bzw. aus einer Harnprobe erhalten wurden, die der Testperson vor Verabreichung von Vitamin C entnommen wurde und nach der Entnahme mit Vitamin C versetzt wurde. Insbesondere gilt für die Cyclovoltagramme 301-305:

**Tabelle 1**

| Cyclovoltagramm | Entnahme |
|---|---|
| 301 | Harnprobenentnahme vor Verabreichung von Vitamin C |
| 302 | Harnprobenentnahme zwei Stunden nach Verabreichung von Vitamin C |
| 303 | Harnprobenentnahme drei Stunden nach Verabreichung von Vitamin C |
| 304 | Harnprobenentnahme fünf Stunden nach Verabreichung von Vitamin C |
| 305 | Harnprobenentnahme vor Verabreichung von Vitamin C mit nachträglicher Hinzufügung von Vitamin C |

Die Cyclovoltagramme 301-305 sind dargestellt, indem entlang der x-Achse 310 die angelegte Spannung in mV und entlang der y-Achse 320 der gemessene Strom in mA aufgetragen ist.

Die Cyclovoltagramme 301-305 zeigen Unterschiede in den Cyclovoltagrammverläufen, die durch die vorliegende Erfindung deutlicher und robuster ausgewertet werden können, wobei es erfindungsgemäß möglich ist, Unterschiede in den Signalen zu erkennen, die einer visuellen Auswertung nicht zugänglich sind.

Die Cyclovoltagramme 301-305 wurden einer Auswertung gemäß den Schritten von Fig. 3 unterzogen. Hierzu wurden vorab Cyclovoltagramme von Harnproben aufgenommen, die Testpersonen entnommen wurden, denen zuvor kein Vitamin C verabreicht wurde. Die aufgenommenen Cyclovoltagramme dieser Harnproben dienten als Referenzproben und wurden verwendet, um eine Abbildungsvorschrift bzw. eine Transformationsmatrix für Meßvektoren von Cyclovoltagrammen zu bilden, wie es im vorhergehenden bezugnehmend auf Fig. 3 beschrieben wurde. Mittels dieser auf diese Art und Weise auf Harnmessungen angepaßten Transformationsmatrix wurden die Meßvektoren bzw. die reduzierten spektralen Meßvektoren der Cyclovoltagramme 301-305 in einen zweidimensionalen Merkmalsraum transformiert.

Fig. 5 stellt den zweidimensionalen Merkmalsraum dar, in den die reduzierten spektralen Meßvektoren von Fig. 4 transformiert wurden. Insbesondere wird der Merkmalsraum von zwei Achsen 400 und 410 aufgespannt, die den zwei Eigenvektoren mit den größten Eigenwerten entsprechen. Die beiden Achsen 400, 410 in Fig. 5 sind derart normiert, daß die Varianz von Merkmalswerten Eins ergibt (Unit Variance). In Anlehnung an die verwendete Hauptkomponentenanalyse, sind die Achsen 400 und 410 mit "Hauptachse 1" bzw. "Hauptachse 2" beschriftet.

Wie es in Fig. 5 zu sehen ist, sind in dem Merkmalsraum fünf Häufungen 301', 302', 303', 304' und 305' von Merkmalspunkten zu erkennen. Jede Häufung 301', 302', 303', 304' und 305' setzt sich aus vier Merkmalspunkten zusammen, die durch die im vorhergehenden erwähnte Hauptkomponententransformation aus den in Fig. 4 gezeigten Cyclovoltagrammen erhalten wurden, indem diese durch eine Gaussverteilung verrauscht wurden. Die Auswertung gemäß der Musterverarbeitung, in diesem Fall der Hauptkomponentenanalyse, ergibt folglich trotz eines Verrauschens der Meßvektoren bzw. der Cyclovoltagramme 301-305 von Fig. 4 mit 10% relativen Rauschens des Maximalwerts eine eindeutige Trennung der verschiedenen Harnproben, wie es in Fig. 5 bei den Häufungen 301'-305' zu sehen ist. Wie bereits erwähnt, sind die Achsen derart normiert, daß die Varianz der Merkmalswerte Eins ergibt. Insbesondere entspricht die Häufung 301' von Merkmalspunkten dem Cyclovoltagramm 301 von Fig. 4, die Häufung 302' von Merkmalspunkten dem Cyclovoltagramm 302 von Fig. 4, usw.

Aufgrund der deutlichen Trennung der Häufungen 301'-305' ist es möglich, weitere Messungen von Harnproben, die Personen unbekanntermaßen entnommen werden, bestimmten Klassen zuzuordnen. Bei dem Beispiel von Fig. 4 und 5 ist beispielsweise bekannt, daß die Probenflüssigkeit des Cyclovoltagramms 301 von Fig. 4 eine Harnprobe war, die einem Patienten vor Verabreichung von Vitamin C entnommen wurde. Es ist ferner bekannt, daß die Probenflüssigkeiten der Cyclovoltagramme 302, 303 und 304 von Fig. 4 Harnproben waren, die einem Patienten nach Verabreichung von Vitamin C entnommen wurden. Schließlich ist außerdem bekannt, daß die Probenflüssigkeit des Cyclovoltagramms 305 von Fig. 4 eine Harnprobe war, die einem Patienten vor Verabreichung von Vitamin C entnommen wurde und nachträglich mit Vitamin C versetzt wurde. Eine erneute Aufnahme und Verarbeitung eines Cyclovoltagramms einer Harnprobe einer unbekannten Testperson kann nun beispielsweise dahingehend ausgelegt werden, daß der Testperson vor Entnahme der Harnprobe entweder kein Vitamin C verabreicht worden war, Vitamin C verabreicht worden war oder zwar kein Vitamin C verabreicht worden war, aber der Harnprobe nachträglich Vitamin C hinzugefügt wurde.

Eine solche qualitative Klassifizierung könnte folgendermaßen durchgeführt werden, indem zunächst der Schwerpunkt der Häufungen 301' von Merkmalspunkten bestimmt wird. Die Bestimmung des Schwerpunkts kann beispielsweise geometrisch erfolgen. Hierauf wird der Schwerpunkt der Häufungen 302', 303' und 304' von Merkmalspunkten bestimmt. Schließlich wird der Schwerpunkt der Häufung 305' ermittelt. Nun wird der Abstand des Merkmalspunkts, der der Harnprobe der unbekannten Testperson zugeordnet ist, zu jedem der drei Schwerpunkte bestimmt. Jeder Abstand kann beispielsweise einer Mahalanobisdistanz entsprechen. Ist der Abstand zu dem Schwerpunkt von 301' am geringsten, so wird rückgeschlossen, daß der Patient vor Entnahme der Harnprobe kein Vitamin C zu sich genommen hat. Ist der Merkmalspunkt dem Schwerpunkt von 302', 303' und 304' am nächsten, so wird rückgeschlossen, daß die Testperson vor der Entnahme der Harnprobe Vitamin C zu sich genommen hat. Falls schließlich der Merkmalspunkt dem Schwerpunkt von 305' am nächsten liegt, wird rückgeschlossen, daß die Testperson vor Entnahme der Harnprobe kein Vitamin C zu sich genommen hat, daß der Harnprobe aber später Vitamin C hinzugefügt worden ist.

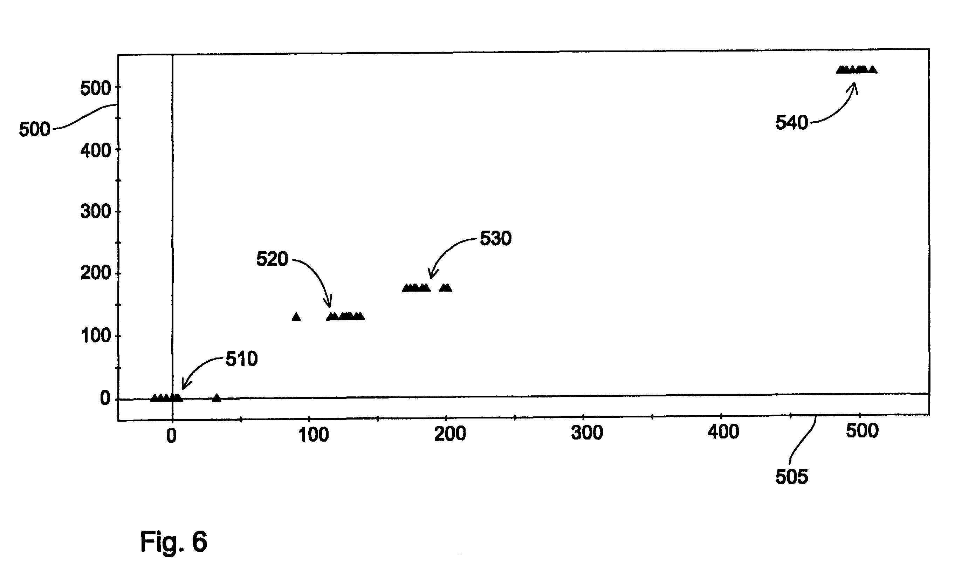

Nachdem in Fig. 5 dargestellt worden ist, wie eine qualitative Zuordnung von Cyclovoltagrammen zu Klassen möglich ist, wird bezugnehmend auf Fig. 6 erläutert, wie aus einem Cyclovoltagramm einer Probenflüssigkeit gemäß dem erfindungsgemäßen Verfahren ein der Probenflüssigkeit zugeordneter, quantitativer Wert erhalten werden kann.

In Fig. 6 ist auf der y-Achse 500 eine Zeitdauer in Minuten aufgetragen, die zwischen einer Verabreichung von Vitamin C und einer Entnahme von Harnproben tatsächlich vergangen ist. Auf einer x-Achse 505 sind die entsprechenden Zeitwerte in Minuten aufgetragen, die sich aus Merkmalspunkten für sieben verrauschte Meßvektoren von vier entsprechenden Harnproben ergaben, wie es im folgenden erläutert wird.

Wie es bereits bezugnehmend auf Fig. 4 und 5 erläutert wurde, wurden die vier Meßvektoren, die sich aus zu verschiedenen Zeitpunkten entnommenen Harnproben ergaben, nach Verrauschen der Meßdaten auf jeweils sieben Merkmalspunkte transformiert, wobei sich vier Häufungen dieser Merkmalspunkte ergaben. Die Entnahmezeitpunkte stellen eine Eigenschaft der Harnproben dar. Die Häufungen von Merkmalspunkten sind den einzelnen Harnproben und folglich den Zeitpunkten ihrer Entnahme zugeordnet. Daraufhin wurde über diese zugeordneten Paare von Häufungen und Zeitwerten linear interpoliert, woraus sich eine Zuordnung ergab, die jedem Punkt in dem Merkmalsraum einen Zeitwert zuordnet. Die in Fig. 6 sichtbaren achtundzwanzig Punkte, die in vier Häufungen 510, 520, 530 und 540 eingeteilt sind, stellen die den jeweiligen Merkmalspunkten zugeordneten Zeitwerte dar. Es zeigt sich, daß trotz Verrauschens der Meßdaten die Zeitdauer zwischen Verabreichung von Vitamin C und Entnahme der Harnprobe relativ genau bestimmt werden kann. Um die Genauigkeit zu verbessern kann statt einer linearen Interpolation eine Interpolation höherer Ordnung verwendet werden. Es können ferner Splines-Funktionen verwendet werden, um zwischen den verschiedenen Merkmalshäufungen zu interpolieren.

In dem vorhergehenden Erwähnten ist gezeigt worden, daß die erfindungsgemäße Vorrichtung bzw. das erfindungsgemäße Verfahren in der Lage sind, verschiedene Charakteristika von Harnproben zu bestimmen. Insbesondere wurde gezeigt, daß sowohl qualitative als auch quantitative Aussagen über eine Harnprobe getroffen werden können.

Es wird jedoch darauf hingewiesen, daß die vorliegende Erfindung ferner auf andere Körperflüssigkeiten, wie z.B. Liquor, Blut, usw., oder auf flüssige Lebensmittel angewendet werden kann. Grundsätzlich ist die vorliegende Erfindung auch auf weitere chemische Lösungen jeglicher Art, sowohl organische als auch anorganische Flüssigkeiten, anwendbar, die erstens ausreichend leitfähig sind, um ein Cyclovoltgramm aufnehmen zu können, und zweitens homogen sind. Insbesondere ist die vorliegende Erfindung folglich auch auf Waschflüssigkeiten für beispielsweise Waschmaschinen und Spülmaschinen (Waschflotte) anwendbar. Ist die zu messende Flüssigkeit nicht ausreichend Leitfähig, so kann eine entsprechende Leitfähigkeit durch Hinzugabe eines Elektrolyten erzielt werden.

Es wird ferner darauf hingewiesen, daß, obwohl im vorhergehenden das Verwenden von lediglich drei Elektroden beschrieben wurde, ferner mehr Elektroden vergesehen sein können, so daß beispielsweise Messungen mit verschiedenen Elektrodenmaterialien gleichzeitig aufgenommen werden können.

Die erforderlichen Berechnungen, die bei den Transformationen bzw. mathematischen Operationen durchgeführt werden müssen, können entweder durch ein Computerprogramm, das auf einem Prozessor ausgeführt wird, einem Anwender-spezifischen IC (ASIC) oder dergleichen ausgeführt werden.

Obwohl im vorhergehenden bezugnehmend auf Fig. 3 beschrieben wurde, daß zur Berechnung der Transformationsmatrix zum Transformieren der Meßvektoren in den Merkmalsraum eine Mehrzahl von Meßvektoren aufgenommen und verwendet werden, kann ferner eine Mehrzahl von Meßvektoren durch Verrauschen eines aufgenommenen Meßvektors, indem derselbe mit einem gaussverteilten Rauschen verrauscht wird, erhalten und verwendet werden.

Ferner wird darauf hingewiesen, daß, obwohl im vorhergehenden beschrieben wurde, daß die Meßvektoren vor der Transformation in den Merkmalsraum durch Anwendung eines mathematischen Operators und ein nachfolgendes Abschneiden von bestimmten Spektralwerten komprimiert werden, es ferner möglich ist, die Hauptkomponentenanalyse direkt auf die Meßvektoren anzuwenden.

Es wird darauf hingewiesen, daß das im vorhergehenden beschriebene Verfahren ein "supervised" Verfahren ist, darin, daß Stützstellen im Merkmalsraum vorliegen, anhand derer eine Interpolation durchgeführt wird, um eine Zurdnung zwischen Merkmalspunkten und Charakteristika zu ermöglichen. Das vorliegende Verfahren ist allerdings auch als "unsupervised" Verfahren implementierbar, wobei hier keine Stützstellen vorliegen, sondern nachträglich über bestimmte Korrelationen eine Kalsseneinteilung hergeleitet wird. Grundsätzlich sind alle multivariaten Signalverarbeitungen anwendbar.

Ein Vorteil des hierin beschriebenen Verfahrens besteht somit darin, daß ein Merkmalsvektor a priori nicht bekannt sein muß. Nach der Bestimmung des Eigensystems, bzw. der Eigenvektoren und der Eigenwerte, kann man a posteriori feststellen, daß die Meßvektoren offenbar bestimmten Eigenschaften im System zugeordnet werden können. Diese Eigenschaften bzw. Klassen können beispielsweise Krankheiten sein. In einem anderen Beispiel können es auch Konzentrationen bestimmter Stoffe sein. Im letzteren Fall ist es natürlich durch den Aufbau eines Modells möglich, einen neuen Meßvektor auf eine bestimmte Konzentration aus einem kontinuierlichen Bereich abzubilden oder, wie es im vorhergehenden erwähnt wurde, auf die Zeitdauer zwischen der Entnahme der Probe und Verabreichung eines Medikamentes abzubilden.

Ferner ist es möglich, anstatt der im vorhergehenden erwähnten Hauptkomponentenanalyse andere Verfahren zu verwenden, um die erhaltenen Meßvektoren in einen niedrigdimensionalen Raum abzubilden. Als Auswertealgorithmen bieten sich die Verfahren der Statistik sowie der neuronalen Netze an. Mit diesen Verfahren können aus den Meßkurven auch solche Merkmale extrahiert werden, die auch für einen geübten Auswerter schwer oder nicht zu erkennen sind. Prinzipiell gesehen handelt es sich bei diesen Algorithmen um Abbildungsvorschriften von einem Koordinatensystem der Meßvektoren in ein anderes, niedrigdimensionales Koordinatensystem von Merkmalen oder physikalischen bzw. chemischen Größen, das die Auswertung enthält. Die Hauptkomponentenanalyse ist ein Beispiel für ein vorteilhaftes Verfahren für diesen Zweck, das aus Meßkurven Merkmale extrahieren kann, die eine Klassifizierung erlauben. Ein wesentlicher Vorteil des Verfahrens besteht darin, daß dasselbe auch als ein "unsupervised" Verfahren ohne Kenntnis der Ergebnisse eingesetzt werden kann, und trotzdem Unterschiede und Klassen in den Proben aufspüren kann, indem beispielsweise a posteriori bestimmte Korrelationen mit bestimmten Charakteristika erfaßt werden. Da es sich um eine Matrizenabbildung handelt, ist das Verfahren linear und robust.

Die Klassen können nicht nur Konzentrationen von einzelnen Stoffen zugeordnet werden, sie können beispielsweise auch bestimmte Krankheitszustände identifizieren, die mit bestimmten Stoffwechselprodukten korreliert sind. Letzteres macht das Verfahren für eine schnelle Analyse von Krankheiten besonders interessant. In dem transformierten Vektorraum der Hauptkomponenten können dann auch Interpolationsverfahren zur Konzentrationsmessung eingesetzt werden, wie beispielsweise in Fig. 6, bzw. die Klasseneinteilung kann als Grundlage für die Methode des sogenannten partial model building eingesetzt werden.

Das hier erörterte Verfahren zur Merkmalsanalyse, d.h. die Hauptkomponentenanalyse, ist jedoch im wesentlichen linear, was es einerseits robust macht, im Fall hoher Nichtlinearität seine Einsatzmöglichkeit jedoch einschränkt. Bei nichtlinearen Zusammenhängen können vorteilhaft Verfahren der künstlichen neuronalen Netze eingesetzt werden, seien es selbstorganisierende Netzwerke (SOM) zur Klassifizierung oder "klassische" neuronale Netze zur Quantifizierung. Die Verfahren der neuronalen Netze sind nicht linear und können somit mehr Anwendungsfälle als lineare Verfahren behandeln, besitzen aber den Nachteil, daß dieselben weniger robust als die Hauptkomponentenanalyse sind. Sie erfordern ebenfalls wegen der größeren Anzahl der Freiheitsgrade einen höheren Kalibrieraufwand, um eine robuste Abbildungsvorschrift zu erreichen.

## Patentansprüche

1. Verfahren zum Bestimmen von Charakteristika einer Probenflüssigkeit mit einer Mehrzahl von Substanzen, das folgende Schritte aufweist:
Aufnehmen (220) von Strom-Spannungs-Meßdaten einer Flüssigkeit mit zumindest einer bekannten Charakteristik,
Transformieren (235) der Meßdaten der Flüssigkeit in einen Merkmalsraum, um eine erste Mehrzahl von Merkmalswerten zu erhalten;
Aufnehmen (240) von Strom-Spannungs-Meßdaten der Probenflüssigkeit;
Transformieren (255) der Meßdaten der Probenflüssigkeit in den Merkmalsraum, um eine zweite Mehrzahl von Merkmalswerten zu erhalten;
Bestimmen (260) von zumindest einer Charakteristik der Probenflüssigkeit basierend auf den Merkmalswerten der Probenflüssigkeit bezogen auf die Merkmalswerte der Flüssigkeit mit der zumindest einen bekannten Charakteristik.

2. Verfahren gemäß Anspruch 1, bei dem die Flüssigkeiten eine Körperflüssigkeit, flüssige Lebensmittel oder Waschflüssigkeiten sind.

3. Verfahren gemäß Anspruch 1 oder 2, bei dem die zumindest eine Charakteristik einer Konzentration der Mehrzahl von Substanzen, einer Krankheitsdiagnoseaussage oder der Zeitdauer zwischen einer Probenentnahme und einer Medikamentverabreichung entspricht.

4. Verfahren gemäß einem der Ansprüche 1 bis 3, bei dem die Schritte des Aufnehmens (220, 240) folgende Schritte aufweisen:
zyklisches Anlegen einer Spannungsrampe an die Flüssigkeit in beiden Richtungen; und
Messen des Elektrolysestroms in Abhängigkeit von der angelegten Spannung.

5. Verfahren gemäß einem der Ansprüche 1 bis 4, das ferner folgende Schritte enthält:
Aufnehmen (200) von Strom-Spannungs-Meßdaten einer Mehrzahl von als Referenzflüssigkeiten vorbestimmten Flüssigkeiten;
Bestimmen (215) einer Transformationsmatrix für die Schritte des Transformierens (235, 255) in den Merkmalsraum.

6. Verfahren gemäß Anspruch 5, bei dem der Schritt des Aufnehmens (200) von Strom-Spannungs-Meßdaten einer Mehrzahl von Referenzflüssigkeiten ferner folgenden Schritt aufweist:
Verrauschen der aufgenommenen Strom-Spannungs-Meßdaten, um weitere Strom-Spannungs-Meßdaten zu erhalten.

7. Verfahren gemäß Anspruch 5 oder 6, bei dem der Schritt des Verrauschens durch Addieren eines gaussverteilten Rauschens zu den Meßdaten durchgeführt wird.

8. Verfahren gemäß einem der Ansprüche 5 bis 7, bei dem der Schritt des Bestimmens (215) der Transformationsmatrix folgende Schritte aufweist:
Bilden einer Kovarianzmatrix aus den Meßdaten der Mehrzahl von Referenzflüssigkeiten;
Berechnen der Eigenwerte und der zugehörigen Eigenvektoren der Kovarianzmatrix; und
Bilden der Transformationsmatrix, derart, daß die Transformationsmatrix eine Abbildungsvorschrift für Meßvektoren in einen Raum liefert, der von den Eigenvektoren aufgespannt wird, deren zugehörigen Eigenwerte einen empirisch vorbestimmten Schwellenwert überschreiten.

9. Verfahren gemäß einem der Ansprüche 1 bis 8, bei dem das Bestimmen (260) von zumindest einer Charakteristik der Probenflüssigkeit folgende Schritte aufweist:
Bestimmen des Abstands der Merkmalswerte der Probenflüssigkeit zu den Merkmalswerten der Flüssigkeit mit der zumindest einen bekannten Charakteristik; und
Zuordnen der zumindest einen bekannten Charakteristik der Flüssigkeit mit der zumindest einen bekannten Charakteristik zu der Probenflüssigkeit, falls der bestimmte Abstand einen bestimmten Grenzwert unterschreitet.

10. Verfahren gemäß einem der Ansprüche 1 bis 8, bei dem Strom-Spannungs-Meßdaten einer Mehrzahl von Flüssigkeiten mit zumindest einer bekannten Charakteristik aufgenommen und transformiert werden, um eine Mehrzahl von Merkmalsvektoren in dem Merkmalsraum zu erhalten.

11. Verfahren gemäß Anspruch 10, bei dem der Schritt des Bestimmens (260) der zumindest einen Charakteristik der Probenflüssigkeit folgende Schritte aufweist:
Bestimmen der Abstände der Merkmalswerte der Probenflüssigkeit zu den Merkmalsvektoren; und
Zuordnen der zumindest einen bekannten Charakteristik der Flüssigkeit mit zumindest einer bekannten Charakteristik, die dem Merkmalsvektor mit dem geringsten Abstand zugeordnet ist, zu der Probenflüssigkeit.

12. Verfahren gemäß Anspruch 10, bei dem die zumindest eine bekannte Charakteristik der Mehrzahl von Flüssigkeiten mit zumindest einer bekannten Charakteristik quantitative Werte sind, die auf eine Eigenschaft bezogen sind, wobei der Schritt des Bestimmens (260) der zumindest einen Charakteristik der Probenflüssigkeit folgende Schritte aufweist:
Interpolieren zwischen den Merkmalswerten und quantitativen Werten der Mehrzahl von Flüssigkeiten mit zumindest einer bekannten Charakteristik, um eine Interpolationsfunktion zu erhalten, die in dem Merkmalsraum definiert ist; und
Zuordnen des Werts der Interpolationsfunktion am Ort der Merkmalswerte der Probenflüssigkeit zu der Probenflüssigkeit.

13. Verfahren gemäß einem der Ansprüche 1 bis 12, das ferner folgenden Schritt aufweist:
Berechnen (205, 225, 245) der Fouriertransformierten der Meßdaten,
wobei die Schritte des Transformierens auf die fouriertransformierten Meßdaten angewendet werden.

14. Verfahren gemäß einem der Ansprüche 1 bis 12, das ferner folgenden Schritt aufweist:
Durchführen einer Wavelet-Transformation der Meßdaten,
wobei die Schritte des Transformierens auf die einer Wavelet-Transformation unterzogenen Meßdaten angewendet werden.

15. Verfahren gemäß Anspruch 13 oder 14, das ferner folgenden Schritt aufweist:
Herausgreifen (210, 230, 250) transformierter Meßdaten, deren Summe einen bestimmten Prozentsatz der Gesamtsumme aller transformierten Meßdaten überschreitet,
wobei die Schritte des Transformierens auf die herausgegriffenen transformierten Meßdaten angewendet werden.

16. Verfahren gemäß einem der vorhergehenden Ansprüche, bei dem ein zur Aufnahme von Strom-Spannungs-Meßdaten verwendetes Elektrodenmaterial für jeden der Schritte (200, 220, 240) des Aufnehmens gleich ist.

17. Verfahren gemäß einem der vorhergehenden Ansprüche, bei dem die Schritte (200, 220, 240) des Aufnehmens mehrmals durchgeführt werden, wobei bei jedem Mal ein zur Aufnahme von Strom-Spannungs-Meßdaten verwendetes Elektrodenmaterial verändert wird, und wobei die mehreren Strom-Spannungs-Meßdaten zusammengefaßt werden.

18. Verfahren gemäß Anspruch 4 oder 16, bei dem eine zur Aufnahme von Strom-Spannungs-Meßdaten verwendete Scangeschwindigkeit für jeden der Schritte (200, 220, 240) des Aufnehmens gleich ist.

19. Verfahren gemäß einem der vorhergehenden Ansprüche, bei dem die Schritte (200, 220, 240) des Aufnehmens mehrmals durchgeführt werden, wobei bei jedem Mal eine zur Aufnahme von Strom-Spannungs-Meßdaten verwendete Scangeschwindigkeit verändert wird, und wobei die mehreren Strom-Spannungs-Meßdaten zusammengefaßt werden.

20. Verfahren gemäß einem der vorhergehenden Ansprüche, das ferner folgenden Schritt aufweist:
vor den Schritten (200, 220, 240) des Aufnehmens, Verdünnen der Flüssigkeiten, bis die Flüssigkeiten einen vorbestimmten Leitfähigkeitswert aufweisen.

21. Verfahren gemäß einem der vorhergehenden Ansprüche, das ferner folgenden Schritt aufweist:
vor den Schritten (200, 220, 240) des Aufnehmens, Einbringen (40) eines Inertgases in die Flüssigkeit, um in der Flüssigkeit gelösten Sauerstoff auszutreiben.

22. Vorrichtung zum Bestimmen von Charakteristika einer Probenflüssigkeit mit einer Mehrzahl von Substanzen, mit folgenden Merkmalen:
einer Aufnahmeeinrichtung zum Aufnehmen von Strom-Spannungs-Meßdaten einer Flüssigkeit mit zumindest einer bekannten Charakteristik und von Strom-Spannungs-Meßdaten der Probenflüssigkeit;
einer ersten Verarbeitungseinrichtung zum Transformieren der Meßdaten der Flüssigkeit in einen Merkmalsraum, um eine erste Mehrzahl von Merkmalswerten zu erhalten, und zum Transformieren der Meßdaten der Probenflüssigkeit in den Merkmalsraum, um eine zweite Mehrzahl von Merkmalswerten zu erhalten; und
einer zweiten Verarbeitungseinrichtung zum Bestimmen von zumindest einer Charakteristik der Probenflüssigkeit basierend auf den Merkmalswerten der Probenflüssigkeit bezogen auf die Merkmalswerte der Flüssigkeit mit der zumindest einen bekannten Charakteristik.

23. Vorrichtung gemäß Anspruch 22, bei der die Flüssigkeiten Körperflüssigkeiten, flüssige Lebensmittel oder Waschflüssigkeiten sind.

24. Vorrichtung gemäß Anspruch 22 oder 23, bei der die zumindest eine Charakteristik einer Konzentration der Mehrzahl von Substanzen, einer Krankheitsdiagnoseaussage oder der Zeitdauer zwischen einer Probenentnahme und einer Medikamentverabreichung entspricht.

25. Vorrichtung gemäß einem der Ansprüche 22 bis 24, bei der die Aufnahmeeinrichtung folgende Merkmale aufweist:
eine Spannungserzeugungseinrichtung zum zyklischen Anlegen einer Spannungsrampe an die Flüssigkeit in beiden Richtungen; und
eine Meßeinrichtung zum Messen des Elektrolysestroms in Abhängigkeit von der angelegten Spannung.

26. Vorrichtung gemäß einem der Ansprüche 22 bis 25, die ferner folgende Einrichtung aufweist:
eine Einrichtung zum Bestimmen einer Transformationsmatrix zur Verwendung bei der Transformation in den Merkmalsraum aus aufgenommenen Strom-Spannungs-Meßdaten einer Mehrzahl von als Referenzflüssigkeiten vorbestimmten Flüssigkeiten.

27. Vorrichtung gemäß einem der Ansprüche 22 bis 26, die ferner folgendes Merkmals aufweist:
eine Einrichtung zum Berechnen der Fouriertransformierten der Meßdaten,
wobei die Einrichtung zum Transformieren die fouriertransformierten Meßdaten transformiert.

28. Vorrichtung gemäß einem der Ansprüche 22 bis 26, die ferner folgendes Merkmal aufweist:
eine Einrichtung zum Durchführen einer Wavelet-Transformation der Meßdaten,
wobei die Einrichtung zum Transformieren die einer Wavelet-Transformation unterzogenen Meßdaten transformiert.

29. Vorrichtung gemäß Anspruch 27 oder 28, die ferner folgendes Merkmal aufweist:
eine Einrichtung zum Herausgreifen transformierter Meßdaten, deren Summe einen bestimmten Prozentsatz der Gesamtsumme aller transformierten Meßdaten überschreitet,
wobei die Einrichtung zum Transformieren die herausgegriffenen transformierten Meßdaten transformiert.

30. Vorrichtung gemäß einem der Ansprüche 22 bis 29, bei der die Aufnahmeeinrichtung folgende Merkmale aufweist:
eine Meßkammer (20);
eine Gegen-, Arbeits- und Referenzelektrode (5, 10, 15), die sich in die Meßkammer (20) erstrecken, wobei an der Referenzelektrode (15) eine feste Referenzspannung anliegt;
eine Spannungserzeugungseinrichtung (25) zum Anlegen einer Spannung zwischen der Gegen- und der Arbeitselektrode (5, 10);
eine Spannungsmeßeinrichtung (25) zum Erfassen der Spannung zwischen der Arbeits- und der Referenzelektrode (10, 15);
eine Strommeßeinrichtung (25) zum Erfassen des Stromes, der zwischen der Arbeitselektrode (10) und der Gegenelektrode (5) fließt.

31. Vorrichtung gemäß Anspruch 30, die ferner folgendes Merkmal aufweist:
eine Einrichtung (30) zum Einbringen eines Inertgases in die Meßkammer (20).

32. Vorrichtung gemäß Anspruch 30 oder 31, bei der die drei Elektroden (5, 10, 15) an einer Sonde angebracht sind, wobei die Sonde auswechselbar ist.

33. Vorrichtung gemäß Anspruch 32, bei der die Sonde folgendes Merkmal aufweist:
eine Einrichtung zum Verstärken des Stromes, der zwischen den Elektroden fließt.

34. Vorrichtung gemäß Anspruch 32 oder 33, bei der die Sonde ferner folgendes Merkmal aufweist:
eine Einrichtung zum Steuern der Temperatur an den Elektroden (5, 10, 15).

35. Vorrichtung gemäß einem der Ansprüche 32 bis 34, bei der die Sonde mehrere Sätze von Elektroden (5, 10, 15) mit unterschiedlichen Materialien aufweist.

36. Vorrichtung gemäß Anspruch 35, bei der das Elektrodenmaterial Gold, Platin oder Graphit ist.
